# EUROPÄISCHE PATENTSCHRIFT

(11) **EP 1 057 474 B1**
(45) Veröffentlichungstag und Bekanntmachung des Hinweises auf die Patenterteilung: **07.08.2002**
(21) Anmeldenummer: 99110457.1
(22) Anmeldetag: 29.05.1999
(51) Int. Cl.: A61K 7/38, C01F 7/48, C01F 7/56

(54) **Verfahren zur Herstellung feinteiliger Antiperspirantwirkstoff Suspensionen**
Method for preparing fine particulated antiperspirant suspensions
Procédé pour la preparation d'agent antitranspirant a fines particules en suspension

(43) Veröffentlichungstag der Anmeldung: 06.12.2000
(73) Patentinhaber: BK Giulini Chemie GmbH & Co. OHG, 67029 Ludwigshafen (DE)
(72) Erfinder: Breker, Johannes, Dr., 67067 Ludwigshafen (DE); Kaufmann, Bruno, 67227 Frankenthal (DE); Reibel, Wolfgang, Dr., 67063 Ludwigshafen (DE); Schanz, Klaus, Dr., 67125 Dannstadt-Schauernheim (DE)

(56) Entgegenhaltungen:
- EP-A- 0 183 171
- EP-A- 0 191 628
- EP-A- 0 256 832
- EP-A- 0 366 230
- EP-A- 0 388 111
- EP-A- 0 412 028
- GB-A- 2 048 229
- US-A- 4 359 456
- US-A- 4 859 446
- US-A- 5 254 230
- US-A- 5 356 609

## Beschreibung

Gegenstand der vorliegenden Erfindung ist ein Verfahren zur Herstellung feinteiliger basisches Aluminiumhalogenid enthaltender Suspensionen, die eine gute Schweißreduktion auf der menschlichen Haut bewirken und darüber hinaus einfach zu handhaben sind.

Im Stand der Technik sind bereits eine Reihe von Verfahren zur Herstellung von Aluminiumhydroxyhalogenid Komplexen, die als Antiperspirant-Wirkstoff verwendet werden können, bekannt. Obwohl die Patente meist die gesamte Palette der Halogenide und weiterer einwertiger nichtkoordinierender Anionen umfassen, haben nur die Chloride eine praktische Bedeutung erlangt.
Neben den Standard Verfahren zur Herstellung der einfachen ca. 50% igen wäßrigen Aluminiumchlorhydrat Lösungen durch Auflösung von Al-Metall in einer unterstöchiometrischen Salzsäuremenge existieren auch Verfahren zur Herstellung sogenannter aktivierter Aluminiumchlorhydrate.
Zur Herstellung der aktivierten Aluminiumchlorhydrate gehen Gosling et al. (US- 4,359,456 ) von Standard Aluminiumchlorhydrat Lösungen aus und führen nach einer Verdünnung bei Temperaturen im Bereich von 50° bis 140°C eine Temperung durch. Diese Temperung führt zu einer Veränderung der Polymerspeziesverteilung. Die höhermolekularen Anteile des Standard Aluminiumchlorhydrats werden dabei zu Gunsten kleinerer Spezies abgebaut, so daß die Lösung keine Spezies größer 100 Å mehr enthält. Die analytische Charakterisierung der so hergestellten Aluminiumchlorhydrat Komplexe wurde mit Hilfe der Ausschlußchromatographie vorgenommen. Mit dieser Methode läßt sich der Anteil der niedermolekularen Komplexe und damit der Aktivierungsgrad verfolgen.
Man hat dabei festgestellt, daß die Aluminiumchlorhydrat Komplexe dann als Antiperspirant besonders wirksam waren, wenn sie hohe Gehalte der Bande III ( relative Retentionszeit ca. 0,79) enthielten.

Ein ähnliches Herstellkonzept verfolgen Fitzgerald et al. (UK-2,048,229 ) durch Alterung von 10 - 25%igen Aluminiumchlorhydrat Lösungen bei 50 bis 80°C für 8 Std. bis 3 Wochen.

Nach beiden alternativen Verfahren ist die durch die Aktivierungsbedingung eingestellte Speziesverteilung in wässriger Lösung nicht beständig, so daß nur durch eine schonende Trocknung ein lagerstabiler effektiver Antiperspirant Wirkstoff erhalten werden kann.

In den Patenten wird als bevorzugte Trocknungsart die Sprühtrocknung beschrieben.

In Antiperspirant Fertigformulierungen werden z.Z. entweder Siebfraktionen der sprühgetrockneten Pulver (z.B. Aerosole ) oder durch Trockenmahlung erhaltene feine Pulver (Roll-on oder Sticks ) eingesetzt.

Es war somit bisher nicht möglich, einen stabilen, effektiven Aluminium - Antiperspirant Wirkstoff in einer flüssigen Form zur Verfügung zu stellen. Die Verarbeitung von pulverförmigen Wirkstoffen, bereitet dem Formulierer aufgrund der Staubbildung jedoch immer große Probleme. Weiterhin kann sogar aufgrund des niedrigen pH-Wertes der (feuchten) Pulver eine Gefährdung der Gesundheit der damit beschäftigten Arbeiter auftreten.

Es bestand somit ein Bedürfnis nach besseren, leichter handhabbaren Antiperspirant-Wirkstoffen, ohne auf die bessere Wirksamkeit der bisherigen Pulverprodukte zu verzichten. Außerdem war nicht möglich, mit den zur Verfügung stehenden pulverförmigen Aluminiumchlorhydrat Komplexe alle Formulierungswünsche der Kosmetikindustrie zu erfüllen, z.B. erwies sich die Herstellung bestimmter halbfester Formulierungen, sog. Soft-Solids als ausgesprochen schwierig.

Es wurde nun überraschenderweise gefunden, daß man besonders gut handhabbare und gleichzeitig effektive Aluminiumhydroxyhalogenid-haltige Antiperspirant-Wirkstoff Suspensionen nach folgendem Verfahren erhält:
Verfahren zur Herstellung von feinteiligen thixotropen und pumpfähigen Antiperspirant Suspensionen, dadurch gekennzeichnet, daß ein als Antiperspirant wirksames Al-Salz, welches durch die folgende Formel beschrieben wird

   Al(OH)_{(3-b)} X_{b} z H₂O

   mit b = 0,4 bis 3, insbesondere mit b=0,4 5 bis 1,0,
   z < 4
   X = Halogen, insbesondere Chlorid,
optional mit einer Puffersubstanz gemischt, unter Ausschluß von Feuchtigkeit in einer nicht wässrigen Ölphase eingemischt und anschließend darin bei Temperaturen unterhalb von 80 °C mit einem Mahlaggregat fein gemahlen wird.

Die Feinmahlung wird bevorzugt in einer Kugelmühle durchgeführt. Dadurch wird in der Suspension eine Feinteiligkeit von 95 % < 20 µm, vorzugsweise < 10 µm, erreicht.

Das als Antiperspirant wirksame Aluminiumsalz weist in einem HPLC Chromatogramm einen Bande III Anteil von > 30 %, insbesondere > 40 % auf.

Durch die direkte Naßmahlung des Sprühproduktes entfällt die aufwendige und staubintensive Pulversiebung und Trockenmahlung und es werden Suspensionen erhalten, die stabiler sind als solche, die durch Aufschlämmen von trockenen gemahlenen Pulver erhalten werden können.

Diese Antiperspirant-Wirkstoffe stellen nicht wässrige Suspensionen dar, die dadurch gekennzeichnet sind, daß die nicht wässrige Phase aus einer im wesentlichen unpolaren, nicht mit Wasser mischbaren organischen Flüssigkeit der Substanzgruppen Alkane, Isoalkane, monofunktionelle Alkohole, polyfunktionelle Alkohole, Fettsäureester von mono- und dibasischen Carbonsäuren mit monofunktionellen und polyfunktionellen Alkoholen, Polyoxyethylenen, Polyoxypropylenen, Polyalkoxylatethern von Alkoholen, cyclischen Silkonen, offenkettigen Silikonen und Kombinationen davon besteht. Insbesondere besteht die nicht wässrige Ölphase aus Silikonöl.

Als Silikonölkomponente werden erfindungsgemäß cyclische Silikone, offenkettige Silikone oder Mischungen davon eingesetzt.

Die erfindungsgemäßen, feinteiligen Antiperspirant - Suspensionen können als Puffersubstanz die Aminosäuren Glycin und/oder Alanin enthalten.

Das Verfahren zur Herstellung von feinteiligen Antiperspirant Suspensionen ist dadurch gekennzeichnet, daß ein als Antiperspirant wirksames Aluminiumsalz, gegebenenfalls in Gegenwart der Aminosäure, bevorzugt Glycin, unter Ausschluß von Feuchtigkeit in einer nicht wässrigen Ölphase gemischt und anschließend vermahlen wird. Als Mahlaggregat wird eine Kugelmühle eingesetzt.

Als ein Antiperspirant wirksames Aluminiumsalz, wird ein basisches Aluminiumhalogenid der folgenden Zusammensetzung eingesetzt:

Al(OH)_{(3-b)}X_{b}zH₂O

mit b = 0,4 bis 3, bevorzugt mit b = 0,45 bis 1,0, z < 4
wobei X = Halogenid, insbesondere Chlorid
wobei das HPLC Chromatogramm des Wirkstoffs bevorzugt einen Bande III Anteil von < 30 %, insbesondere > 40 % aurweist.

Im Falle der eine Aminosäure enthaltenden Variante, wird ein als Antiperspirant wirksames Aluminiumsalz, ein basisches Aluminiumhalogenid der folgenden Zusammensetzung eingesetzt:

Al (OH)_{(3-b)} X _{b} (Aminosäure) z H₂O

mit X = Halogen, insbesondere Chlorid,
b = 0,4 bis 3, bevorzugt mit b = 0,45 bis 1,0
z<2
und dem Molverhältnis Aminosäure zu Aluminium von 0 bis 0,33.

Die Mahlung der Antiperspirant Suspension beim erfindungsgemäßen Verfahren ist dadurch gekennzeichnet, daß dieser Vorgang bei Temperaturen unterhalb von 60 °C, insbesondere unterhalb von 40 °C durchgeführt wird.
Die Antiperspirant - Suspensionen werden mit Vorteil in kosmetischen, nicht wässrigen Formulierungen, z. B. in sog. soft - solids eingesetzt.

### Die nachfolgenden Beispiele verdeutlichen die Erfindung näher:

### Beispiele für erfindungsgemäße Suspensionen

### Beispiel 1

In einem 400 l Reaktor mit Propellerrührwerk werden 130 kg Cyclomethicone ( DC 345,von der Fa. Dow - Corning) vorgelegt. Unter Rühren werden 159 kg eines aktivierten Aluminiumchlorhydrat Pulvers mit einem Aluminium - Gehalt von 26,0% und einem Chlorid Gehalt von 17,0%, hergestellt nach Pat. US 4.359.456.

Die homogene Suspension mit einem Feststoffgehalt von 55 % wurde anschließend mit einer Kugelmühle (Fa. Fryma - CoBall-Mill ) auf eine Endfeinheit von 99,9% < 12µm und 90% < 9 µm feinst vermahlen. Die Austrittstemperatur der Produktsuspension lag dabei bei 25 °C - 30 °C . Die dabei erhaltene thixotrope und pumpfähige Suspension ist stabil gegenüber Sedimentation.

### Beispiel 2 (erfindungsgemäß)

In einem 100 1 Reaktor mit Propellerrührwerk werden 22,5 kg Cyclomethicone (DC 345, von der Fa. Dow - Coming) vorgelegt. Unter Rühren werden 22,46 kg eines aktivierten Aluminiumchlorhydrat Pulvers mit einem Aluminium - Gehalt von 26 % und einem Chlorid - Gehalt von 17,1%, hergestellt nach Pat. US 4.359.456 und 5,04 kg Glycin eingetragen.

Die homogene Suspension mit einem Feststoffgehalt von 55 % wurde anschließend mit einer Kugelmühle (Fa. Fryma - CoBall-Mill ) auf eine Endfeinheit von 99 % < 18 µm und 90 % < 9 µm sehr fein vermahlen. Die Austrittstemperatur der Produktsuspension lag dabei bei 25 °C - 30 °C . Die dabei erhaltene thixotrope und pumpfähige Suspension ist stabil gegenüber Sedimentation.

### Beispiel 3

In einem 1001 Reaktor mit Propellerrührwerk werden 20 kg Cyclomethicone (DC 345 von der Fa. Dow-Corning) vorgelegt. Unter Rühren werden 30 kg eines aktivierten Aluminiumchlorohydrat Pulvers mit einem Aluminiumgehalt von 26 % und einem Chloridgehalt von 17,1 % hergestellt nach Pat US 4.359.456, eingetragen. Die homogene Suspension mit einem Feststoffgehalt von 60 % wurde anschließend mit eienr Kugelmühle (Fa. Fryma - CoBall-Mill) auf eine Endfeinheit von 99 % < 71 µm vermahlen. Die Austrittstemperatur der Produktsuspension lag dabei bei 25 °C - 30 °C. Die dabei erhaltene thixotrope und pumpfähige Suspension ist stabil gegenüber Sedimentation.

### Vergleichsbeispiel

In einem 101 Behälter mit Dispenserrührwerk werden 2220 g Cyclomethicone (DC 345 Dow-Corning) vorgelegt. Unter Rühren werden 2750 g eines aktivierten Aluminiumchlorhydrat Pulvers mit einem Aluminiumgehalt von 26 % und einem Chlorid Gehalt von 17 %, hergestellt nach US 4,359,456, welches mittels Luftstrahlmühle auf eine Feinheit von 99 % <10 µm gemahlen wurde, eingetragen. Die homogene Suspension mit einem Feststoffgehalt von 55 % war dünnflüssig und nicht sedimentationsstabil.

Nachfolgend werden die erfindungsgemäßen Suspension zur nicht limitierenden Veranschaulichung in kosmetischen Formulierungen eingebracht:

### Roll-on Formulierung

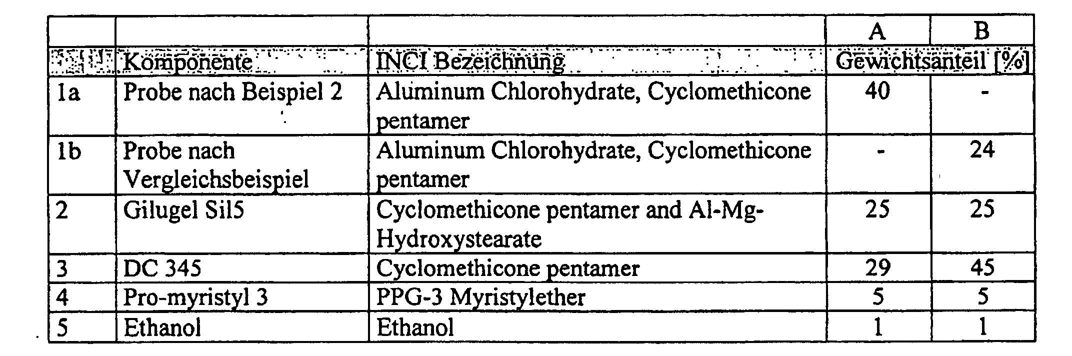

Die Komponenten 2, 3, 4 und 5 werden gemischt und homogenisiert. Die Komponente 1 wird anschließend eingerührt.

Die Formulierung A zeigt eine verbesserte Sedimentationsstabilität bei Lagerung gegenüber Formulierung B.

### Antiperspirant Aerosol Formulierung (masterbatch)

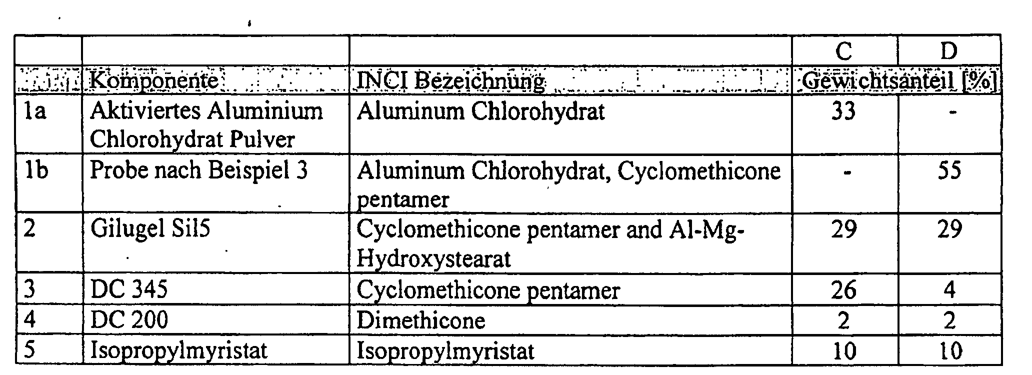

Die Komponenten 2, 3, 4 und 5 werden gemischt und homogenisiert. Die Komponente 1 wird anschließend eingerührt.
Die Formulierung D zeigt eine verbesserte Sedimentationsstabilität bei Lagerung gegenüber Formulierung C.
Für die Abfüllung in Aerosoldosen wird 1 Gewichtsteil der Masterbatch Formulierung mit 3 Gewichtsteilen des Treibgases (Propan, Butan, n-Pentan ) gemischt.

## Patentansprüche

1. Verfahren zur Herstellung von feinteiligen, thixotropen und pumpfähigen Antiperspirant Suspensionen **dadurch gekennzeichnet, daß** ein als Antiperspirant wirksames Al-Salz, welches durch die folgende Summenformel beschrieben wird
Al (OH)_{(3-b)} X_{b} z H₂O
mit b = 0,4 bis 3, insbesondere mit b = 0,45 bis 1,0,
z<4,
X = Halogen, insbesondere Chlorid,
optional mit einer Puffersubstanz gemischt, unter Ausschluß von Feuchtigkeit in einer nicht wässrigen Ölphase eingemischt und anschließend darin bei Temperaturen unterhalb von 80°C mit einem geeigneten Mahlaggregat fein gemahlen wird.

2. Verfahren zur Herstellung von feinteiligen Antiperspirant Suspensionen nach Anspruch 1 **dadurch gekennzeichnet, daß** das als Antiperspirant wirksames Al-Salz im HPLC einen Bande III Anteil von > 30%, bevorzugt > 40% aufweist.

3. Verfahren zur Herstellung von feinteiligen Antiperspirant - Suspensionen nach den Ansprüchen 1 und 2 **dadurch gekennzeichnet, daß** die nicht wässrige Phase aus einer unpolaren, nicht mit Wasser mischbaren organischen Flüssigkeit der Substanzgruppen Alkane, Isoalkane, monofunktionelle Alkohole, polyfunktionelle Alkohole, Fettsäureester von mono- und dibasischen Carbonsäuren mit monofunktionellen und polyfunktionellen Alkoholen, Polyoxyethylenen, Polyoxypropylenen, Polyalkoxylatethern von Alkoholen, cyclischen Silkonen, offenkettigen Silikonen und Kombinationen davon besteht.

4. Verfahren zur Herstellung von feinteiligen Antiperspirant - Suspensionen nach den Ansprüchen 1 und 2 **dadurch gekennzeichnet, daß** als Silikonölkomponente cyclische Silikone, offenkettige Silikone oder Abmischungen davon eingesetzt werden.

5. Verfahren zur Herstellung von Antiperspirant Suspensionen nach Anspruch 1-4 **dadurch gekennzeichnet, daß** die Feinmahlung auf einer Kugelmühle durchgeführt wird.

6. Verfahren zur Herstellung von Antiperspirant Suspensionen nach den Ansprüchen 1 bis 5, **dadurch gekennzeichnet, daß** eine Feinteiligkeit der Suspension von 95% < 20µm erreicht wird.

7. Verfahren zur Herstellung von Antiperspirant Suspensionen nach den Ansprüchen 1 bis 6, **dadurch gekennzeichnet, daß** eine Feinteiligkeit der Suspension von 95% < 10µm erreicht wird.

8. Verfahren zur Herstellung von Antiperspirant Suspensionen nach den Ansprüchen 1 bis 7, **dadurch gekennzeichnet, daß** die Feinmahlung bei Temperaturen unterhalb von 60°C, bevorzugt unterhalb von 40°C durchgeführt wird.

9. Verfahren zur Herstellung von Antiperspirant Suspensionen nach den Ansprüchen 1 bis 8, **dadurch gekennzeichnet, daß** als Puffersubstanz eine Aminosäure, bevorzugt die Aminosäure Glycin zugesetzt wird.

10. Schweißhemmende kosmetische Zusammensetzung, **dadurch gekennzeichnet, daß** sie als Wirkkomponente eine Antiperspirant Suspension, welche nach dem Verfahren der Ansprüche 1 bis 9 hergestellt wird, enthält.

## Claims

1. Process for the synthesis of finely divided thixotropic antiperspirant suspensions capable of being pumped, **characterised in that** an Al salt effective as an antiperspirant, which is described by the following empirical formula
Al(OH)_{(3-b)}X_{b} zH₂O
where b = 0.4 to 3, and particularly b = 0.45 to 1.0
z < 4,
and X = a halogen, especially chlorine,
is optionally be mixed with a buffer substance, with exclusion of humidity, into a non-aqueous oil phase and subsequently finely ground therein with a suitable milling device at temperatures below 80° C to the required fineness.

2. Process for the synthesis of finely divided antiperspirant suspensions according to Claim 1, **characterised in that** the Al salt effective as an antiperspirant exhibits a band III proportion of > 30 %, and preferably > 40 % in an HPLC.

3. Process for the synthesis of finely divided antiperspirant suspensions according to Claims 1 and 2, **characterised in that** the non-aqueous phase consists of a nonpolar organic fluid not miscible with water, of the substance groups alkanes, isoalkanes, monofunctional alcohols, polyfunctional alcohols, fatty acid esters of monobasic and dibasic carboxylic acids with monofunctional and polyfunctional alcohols, polyoxyethylenes, polyoxypropylenes, polyalkoxylate ethers of alcohols, cyclic silicones, open-chained silicones and combinations thereof.

4. Process for the synthesis of finely divided antiperspirant suspensions according to Claims 1 and 2, **characterised in that** as silicone oil components, cyclic silicones, open-chain silicones or mixtures thereof are used.

5. Process for the synthesis of antiperspirant suspensions according to Claim 1 - 4, **characterised in that** the fine grinding is carried out in a ball mill.

6. Process for the synthesis of antiperspirant suspensions according to the Claims 1 to 5, **characterised in that** a fineness of the suspension of 95 % < 20 µm is achieved.

7. Process for the synthesis of antiperspirant suspensions according to the Claims 1 to 6, **characterised in that** a fineness of the suspension of 95 % < 10 µm is achieved.

8. Process for the synthesis of antiperspirant suspensions according to the Claims 1 to 7, **characterised in that** the fine grinding is carried out at temperatures below 60° C, and preferably below 40 ° C.

9. Process for the synthesis of antiperspirant suspensions according to the Claims 1 to 8, **characterised in that** as the buffer substance an amino acid, and preferably the amino acid glycine is added.

10. Perspiration-inhibiting cosmetic preparation, **characterised in that** as active ingredient it contains an antiperspirant suspension synthesised according to one of the Claims 1 to 9.

## Revendications

1. Méthode de fabrication de suspensions antisudorales thixotropes et capables d'être pompées en fines particules **caractérisées par le fait qu'**un sel d'aluminium à action sudorale décrit par la formule brute suivante
Al (OH)_{(3-b)}X_{b} z H₂O
avec b = 0,4 à 3, surtout avec b = 0,45 à 1,0,
z < 4,
X = halogène, surtout du chlorure,
est mélangé en option à une substance tampon et homogénéisé à l'exclusion de l'humidité dans une phase huileuse non aqueuse puis finement titruré dans un broyeur approprié à des températures inférieures à 80 °C.

2. Méthode de fabrication de suspensions antisudorales en fines particules selon la revendication 1, **caractérisée par le fait que** le sel d'aluminium, actif en tant qu'antisudoral, présente, dans un chromatogramme HPLC, une part de bande III > 30 %, plus particulièrement > 40 %.

3. Méthode de fabrication de suspensions antisudorales en fines particules selon la revendication 1 et 2, **caractérisée par le fait que** la phase non aqueuse est constituée par une substance organique non polaire et non miscible à l'eau des groupes des substances alcanes, isoalcanes, alcools monofonctionnels, alcools polyfonctionnels, esters d'acides gras d'acides carboniques monobasiques et dibasiques avec des alcools monofonctionnels et polyfonctionnels, polyoxyéthylènes, polyoxypropylènes, polyalcoxylathème d'alcools, silicones cycliques, silicones à chaînes ouvertes et de combinaisons de ceux-ci. La phase huileuse non aqueuse est composée essentiellement d'huile silicone.

4. Méthode de fabrication de suspensions antisudorales en fines particules selon la revendication 1 et 2, **caractérisée par le fait qu'**en tant que composant d'huile silicone, il est utilisé des silicones cycliques, des silicones à chaîne ouverte ou leurs mélanges.

5. Méthode de fabrication de suspensions antisudorales selon la revendication 1 - 4, **caractérisée par le fait que** la titruration fine s'opère dans un titrurateur à boulets.

6. Méthode de fabrication de suspensions antisudorales selon les revendications 1 - 5, **caractérisée par le fait que** l'on obtient une finesse de particules de la suspension de 95 % < 20 µm.

7. Méthode de fabrication de suspensions antisudorales selon les revendications 1 - 6, **caractérisée par le fait que** l'on obtient une finesse de particules de la suspension de 95 % < 10 µm.

8. Méthode de fabrication de suspensions antisudorales selon les revendications 1 - 7, **caractérisée par le fait que** la titruration fine est opérée à des températures inférieures à 60 °C, de préférence en dessous de 40 °C.

9. Méthode de fabrication de suspensions antisudorales selon les revendications 1 - 8, **caractérisée par le fait qu'**en tant que substance tampon, il y a adjonction d'un acide aminé, de préférence l'acide aminé glycine

10. Composition cosmétique sudoinhibitrice **caractérisée par le fait qu'**en tant que composant actif, elle contient une suspension antisudorale fabriquée selon la méthode des revendications 1 à 9.
